# EUROPÄISCHE PATENTSCHRIFT

(11) **EP 1 733 676 B1**
(45) Veröffentlichungstag und Bekanntmachung des Hinweises auf die Patenterteilung: **01.08.2012**
(21) Anmeldenummer: 05013063.2
(22) Anmeldetag: 17.06.2005
(51) Int. Cl.: A61B 5/00, A61B 5/145, A61M 5/172, A61B 5/1486

(54) **Sensorsystem sowie Anordnung und Verfahren zur Überwachung eines Inhaltsstoffs, insbesondere Glucose in Körpergewebe**
Sensor system, arrangement and method for monitoring a compound, in particular glucose in body tissue.
Système de capteur, disposition et méthode pour surveiller un composé, en particulier le glucose dans le tissu du corps.

(43) Veröffentlichungstag der Anmeldung: 20.12.2006
(60) Teilanmeldung: 10181161.0
(73) Patentinhaber: F. Hoffmann-La Roche AG, 4070 Basel (CH); Roche Diagnostics GmbH, 68305 Mannheim (DE)
(72) Erfinder: Ocvirk, Gregor, 68239 Mannheim (DE); Rinne, Helmut, 22587 Hamburg (DE); Staib, Arnulf, 64646 Heppenheim (DE)
(74) Vertreter: Pfiz, Thomas

(56) Entgegenhaltungen:
- US-A- 5 586 553
- US-A1- 2005 033 133
- US-B1- 6 558 351

## Beschreibung

Die Erfindung betrifft ein Sensorsystem, insbesondere zur Glucoseüberwachung, mit einem zumindest partiell in Körpergewebe, vorzugsweise Unterhautgewebe einsetzbaren Träger, einem an dem Träger angeordneten Fluidkanal zum Durchleiten einer Flüssigkeit und einem auf einen Inhaltsstoff der Flüssigkeit und/oder des Körpergewebes ansprechenden Sensor. Die Erfindung betrifft weiter eine Anordnung sowie ein Verfahren zur Überwachung eines Inhaltsstoffs, insbesondere Glucose in Körpergewebe unter Verwendung eines Sensorsystems.

Körpergewebe bestehen aus Zellen und einer Gewebestruktur in einer Flüssigkeitsumgebung, in der Stoffwechselprodukte zwischen den Zellen und den Blutgefäßen transportiert werden. Für eine Glucose-Überwachung speziell von Diabetes-Patienten kann eine Sonde längere Zeiträume in Gewebe eingesetzt werden, um über Diffusionsvorgänge Inhaltsstoffe aus der Gewebeflüssigkeit kontinuierlich zu gewinnen und aus dem Effusat den Glucosegehalt im Gewebe zu bestimmen. Dieser kann eng mit dem Blutglucosegehalt korreliert werden, ohne dass ein invasiver Zugang zum Blutkreislauf erforderlich wäre. Zugleich ist es möglich, abhängig von der erfassten Glucosekonzentration eine insulinhaltige Medikationslösung zuzuführen, um so eine Art künstlichen Pankreas zu schaffen. Bei bekannten Systemen mit Wirkstoffdosierung ist der Aufbau sehr komplex und das in das Körpergewebe eingesetzte System ist nicht in integrierter und miniaturisierter Bauweise realisiert. Insbesondere besteht bei starren Nadelsensoren die Gefahr, dass während des Verbleibs im Körper zusätzliche Verletzungen des umgebenden Gewebes und als Folge hiervon signifikante Fehler in der Bestimmung der Analytkonzentration auftreten.

Ausgehend hiervon liegt der Erfindung die Aufgabe zugrunde, die im Stand der Technik aufgetretenen Nachteile zu vermeiden und bei einer implantierbaren Vorrichtung und einem entsprechenden Verfahren die Bestimmung eines Inhaltsstoffs in der umgebenden Matrix und das Durchleiten einer Flüssigkeit auf einfache Weise zu ermöglichen.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen. Ein System und ein Verfahren gemäß dem Oberbegriff des unabhängigen Ansprüche ist aus Dokument US-A-5 304 183 bekannt.

Die Erfindung geht von dem Gedanken aus, einen Sensor und eine gesonderte Flüssigkeitspassage auf einem Substrat zu integrieren. Dementsprechend wird erfindungsgemäß vorgeschlagen, dass der Sensor für einen direkten Kontakt mit dem Körpergewebe getrennt von dem Fluidkanal an dem Träger angeordnet ist. Dadurch ist es möglich, außerhalb der ab- bzw. zugeführten Flüssigkeit Informationen über einen Inhaltsstoff direkt aus dem Gewebe zu gewinnen. Auf diese Weise kann die Antwortzeit und insgesamt das Zeitverhalten des Systems optimiert werden, während aufgrund der integrierten Bauweise auch die Herstellung vereinfacht wird. Zudem entfällt auch die Bevorratung, der Transport und die Sammlung von Perfusionsflüssigkeiten, so dass sich die Systemgröße insbesondere für am Körper tragbare Applikationen stark reduzieren lässt.

Der Sensor steht im Abstand von einer gewebeseitigen Öffnung des Fluidkanals in Eingriff mit dem Körpergewebe. Dadurch lassen sich verschiedene Applikationen auf einfache Weise realisieren, wobei nur eine Einstichstelle notwendig ist.

Eine außerhalb des Körpergewebes befindliche Kalibriereinrichtung für den Sensor ist über den Fluidkanal mit interstitieller Flüssigkeit beaufschlagbar ist, wobei die Kalibriereinrichtung einen Referenzsensor zum Kalibrieren der Ausgangssignale des Sensors aufweist.

Der Fluidkanal ist an eine Pumpeinheit zur Entnahme von interstitieller Flüssigkeit aus dem Körpergewebe angeschlossen .

Eine weitere Applikationsmöglichkeit besteht darin, dass der Fluidkanal an eine Dosiereinheit zum Einleiten einer insbesondere insulinhaltigen Medikationsflüssigkeit in das Körpergewebe angeschlossen ist.

Ferner ist es vorteilhaft, wenn der Fluidkanal an eine Fördereinheit zum Einleiten einer Kalibrierlösung bekannter Zusammensetzung in das Körpergewebe angeschlossen ist.

Für eine direkte Messwerterfassung ist es günstig, wenn der Sensor einen in Berührung mit dem Körpergewebe bringbaren aktiven Sensorbereich aufweist.

Eine vorteilhafte Ausführung sieht vor, dass der Sensor zur vorzugsweise kontinuierlichen Erfassung von Glucose als Inhaltsstoff in der interstitiellen Flüssigkeit ausgebildet ist.

Eine weitere Verbesserung wird dadurch erreicht, dass der Sensor in Mehrelektrodenausführung mehrere auf einer in das Körpergewebe eingreifenden Oberfläche des Trägers angeordnete Elektroden aufweist.

Um den Tragekomfort für den Probanden zu erhöhen und zusätzliche Verletzungen des umgebenden Gewebes während des Tragens zu vermeiden, ist es vorteilhaft, wenn der Träger durch ein flexibles Substrat gebildet ist, wobei der Fluidkanal und der Sensor in dem Substrat integriert sind.

Vorteilhafterweise ist der vorzugsweise biegsame und stumpfkantige Träger über Einführhilfsmittel in das Körpergewebe einsetzbar.

Für die Flüssigkeitsabfuhr und -zufuhr wird vorgeschlagen, dass der Fluidkanal mindestens eine gewebeseitige Öffnung, mindestens eine außerhalb des Körpergewebes befindliche Öffnung und mindestens einen längsseitig geschlossenen oder halboffenen, insbesondere kapillaren Fließpfad zwischen den Öffnungen aufweist.

Herstellungstechnisch ist es von Vorteil, wenn der Fluidkanal vorzugsweise durch Laserbearbeitung, Heißprägen oder Photolithographie in den gegebenenfalls mehrteiligen Träger integriert ist.

Eine weitere Verbesserung wird dadurch erreicht, dass der Fluidkanal einen Durchflussquerschnitt von weniger als 1 mm² aufweist.

Je nach Anwendung ergeben sich vorteilhafte Ausgestaltungen dadurch, dass der Fluidkanal auf einer den Sensor tragenden Fläche des Trägers im Erfassungsbereich des Sensors mündet, oder dass der Fluidkanal an einer von dem Sensor abgewandten oder entfernten Partie des Trägers außerhalb des Erfassungsbereichs des Sensors mündet.

Um eine Bestimmung eines Inhaltsstoffs an zwei unterschiedlichen subkutanen Messstellen zu ermöglichen, können als Sensor mindestens zwei in unterschiedlichem Abstand von einer gewebeseitigen Öffnung des Fluidkanals befindliche Einzelsensoren vorgesehen sein.

In vorteilhafter Ausgestaltung weist der Sensor einen Einzelsensor für einen Analyten und einen Einzelsensor für einen über den Fluidkanal in das Körpergewebe einleitbaren Wirkstoff auf.

Gegenstand der Erfindung ist auch eine Anordnung zur Kontrolle eines Inhaltsstoffs, insbesondere Glucose in Körpergewebe, mit einem oben beschriebenen Sensorsystem. Hierbei ist es vorgesehen, dass das Sensorsystem mit einer Signalverarbeitungseinheit zur quantitativen Bestimmung des Inhaltsstoffs gekoppelt ist. Eine weitere vorteilhafte Kombinationswirkung wird dadurch erreicht, dass die durch den Fluidkanal hindurchgeleitete, insbesondere insulinhaltige Flüssigkeitsmenge mittels einer Steuer- oder Regeleinrichtung kontrollierbar ist.

In verfahrensmäßiger Hinsicht wird die eingangs genannte Aufgabe dadurch gelöst, dass der Sensor an dem Träger getrennt von dem Fluidkanal angeordnet und in direkten Kontakt mit dem Körpergewebe gebracht wird.

Um eine Signaldrift zu kompensieren, wird über den Fluidkanal interstitielle Flüssigkeit entnommen und der Inhaltsstoff mittels eines Referenzsensors erfasst, und wird der in Kontakt mit dem Körpergewebe befindliche Sensor nach Maßgabe des Referenzsensors kalibriert. Hier sollte die Kalibrierung in gegebenen Zeitintervallen automatisch durchgeführt werden.

Im Folgenden wird die Erfindung anhand der in der Zeichnung schematisch dargestellten Ausführungsbeispiele näher erläutert. Es zeigen
- Fig. 1: ein Blockschaltbild einer Anordnung zur Glucosekontrolle in Körpergewebe;
- Fig. 2: ein Sensorsystem der Anordnung nach Fig. 1 in der Draufsicht; und
- Fig. 3 bis 5: Schnitte entlang den Linien 3-3, 4-4 und 5-5 der Fig. 2.

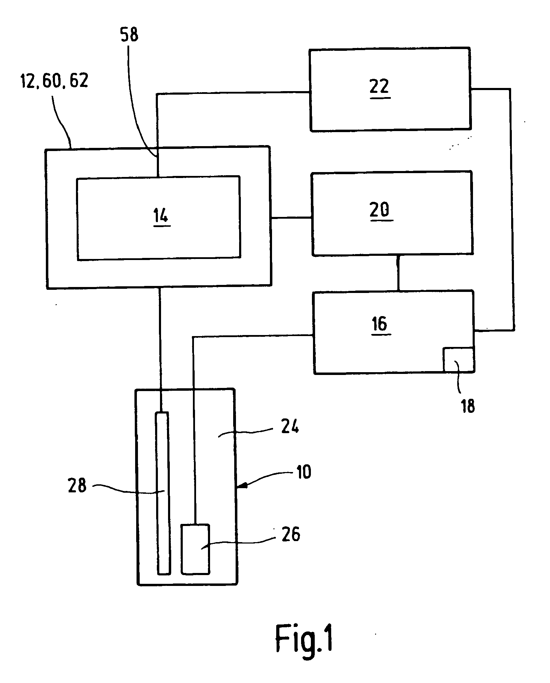

Das in Fig. 1 gezeigte Monitoring-System umfasst ein Sensorsystem 10, eine Fördereinheit 12 mit Flüssigkeitsreservoir 14, eine Signalverarbeitungseinheit 16 mit Alarmgeber 18, eine Steuereinrichtung 20 für die Fördereinheit 12 und einen Kalibriereinrichtung 22. Die Anordnung ermöglicht mittels des subkutan implantierbaren Sensorsystems 10 die Bestimmung von Inhaltsstoffen (Glucose) in der umgebenden Matrix und das Durchleiten einer Flüssigkeit.

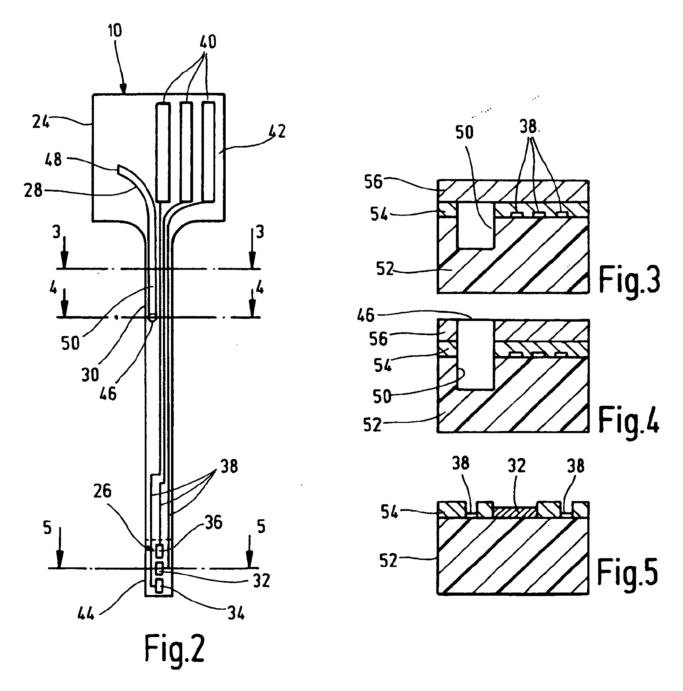

Wie in Fig. 2 gezeigt, weist das Sensorsystem 10 einen Träger 24, einen darauf aufgebrachten elektrochemischen Sensor 26 sowie einen integrierten Fluidkanal 28 auf. Der flexible Träger 24 ist beispielsweise aus einem biegsamen Folienmaterial als Substrat gebildet und zumindest in einem Schaftbereich 30 in das Körpergewebe implantierbar. Zu diesem Zweck wird ein nicht gezeigtes Einführhilfsmittel eingesetzt, welches die erforderliche Gewebeöffnung schafft. Grundsätzlich ist es auch möglich, dass der Träger aus einem biegesteifen Material nadelförmig ausgebildet ist und direkt in das Körpergewebe eingestochen werden kann.

Der Sensor 26 umfasst eine Arbeitselektrode 32, eine Gegenelektrode 34 sowie eine Referenzelektrode 36. Die Elektroden stehen über jeweilige Leiterbahnen 38 in elektrisch leitender Verbindung mit zugeordneten Kontaktzungen 40, welche an einem außerhalb des Körpergewebes befindlichen Anschlussteil 42 des Trägers 24 angeordnet sind.

Die Arbeitselektrode 32 zeichnet sich als Enzymelektrode dadurch aus, dass durch Umsetzung des Analyten (Glucose) mit einem spezifischen Enzym ein Zwischenprodukt gebildet wird, welches auf der Arbeitselektrode durch Anlegen eines konstanten Potentials zwischen Referenz- und Arbeitselektrode elektrochemisch umgesetzt wird. Dadurch kann in an sich bekannter Weise ein mit der Glucosekonzentration korreliertes Messsignal erfasst werden. Über die Referenzelektrode 36 kann das Potential zwischen Referenz- und Arbeitselektrode mittels eines Potentiostaten konstant gehalten werden, so dass die elektrochemische Umsetzung von Interferenten unterbunden werden kann.

Die Arbeitselektrode 32 kann mit zusätzlichen Schichten versehen sein, welche als Diffusionshemmschicht für Glucose, als Interferentensperrschicht und als gewebszugewandte biokompatible Schicht dienen. Zweckmäßig ist die Größe der Gegenelektrode 34 so zu wählen, dass die Stromdichte und somit die auftretende Überspannung minimiert wird. Die Form der Elektroden ist nicht eingeschränkt, vorzugsweise kommen rechteckige oder runde Elektrodenformen zum Einsatz. Möglich ist es auch, dass die Elektroden ineinander greifen. In jedem Fall bilden die Elektroden mit ihrer freien Oberfläche einen in direkte Berührung mit dem Körpergewebe bringbaren aktiven Sensorbereich 44, welcher räumlich getrennt von dem Fluidkanal 28 auf dem Träger 24 angeordnet ist. Denkbar ist es auch, dass mehrere Einzelsensoren als Mehrelektrodensysteme auch über gemeinsame Elektroden verfügen.

Durch den Fluidkanal 28 kann eine Flüssigkeit dem Körpergewebe entnommen oder diesem zugeführt werden. Zu diesem Zweck weist der Fluidkanal 28 mindestens eine gewebeseitige Öffnung 46, mindestens eine außerhalb des Körpergewebes befindliche Öffnung 48 und mindestens einen Fließpfad 50 zwischen den Öffnungen auf. Der Fließpfad 50 ist zweckmäßig seitlich geschlossen und kann einen kapillaraktiven Querschnitt beispielsweise von weniger als 1 mm² besitzen. Damit wird auch das in dem Fluidkanal 28 enthaltene Flüssigkeitsvolumen gering gehalten.

Die gewebeseitige Öffnung 46 sollte so ausgeführt sein, dass durch eintretende Körperreaktionen in diesem Bereich keine im Verhältnis zum Fließpfad 50 signifikante Erhöhung des hydrodynamischen Widerstands über die Implantationszeit feststellbar ist. Hierzu ist es vorteilhaft, die Oberfläche rund um diese Öffnung 46 so auszuführen, dass die Permeabilität für den Analyten bzw. für den jeweilig zugeführten Wirkstoff über die Zeit der Implantation hinweg im Wesentlichen unverändert bleibt. Hierzu ist die Oberfläche hinsichtlich der physikalischen und chemischen Eigenschaften, der Mikrostruktur und der Morphologie so zu wählen, dass die Protein- und Zelladsorption gering gehalten wird. Hinsichtlich der chemischen Beschaffenheit der Oberfläche ist unter anderem eine geringe Oberflächenladung besonders vorteilhaft.

Wie in Fig. 3 bis 5 gezeigt, kann der Träger 24 mehrlagig zusammengesetzt sein und insbesondere aus einer Basisfolie 52, einer Isolierschicht 54 und einer Decklage 56 bestehen. Der Fließpfad 50 bzw. die Flüssigkeitspassage wird vorzugsweise durch Laserablation, Heißprägen oder Photolithographie erzeugt und durch die Deckschicht 56 mit Ausnahme der Öffnungen 46, 48 flüssigkeitsdicht verschlossen.

In der gezeigten Ausführung ist der subkutan implantierbare Glucosesensor 26 über die Kontaktzungen 40 an die extrakorporale Signalverarbeitungseinheit 16 angeschlossen, um ein mit dem Glucosegehalt der interstitiellen Flüssigkeit und damit auch mit der Blutglucose korreliertes Messergebnis für den Benutzer anzuzeigen und gegebenenfalls über den Alarmgeber 18 einen Alarm auszulösen, wenn der normoglykämische Bereich verlassen wird.

Für eine automatische Selbstkalibrierung kann über den Fluidkanal 28 und die nachgeordnete Fördereinheit 12 interstitielle Flüssigkeit entnommen werden, so dass deren Glucosegehalt mittels der externen Kalibriereinrichtung 22 parallel bestimmt werden kann. Zu diesem Zweck weist die Kalibriereinrichtung 22 einen Referenzsensor 58 auf, welcher mit der Gewebeflüssigkeit beaufschlagbar ist. Damit ist es möglich, dass in gegebenen Zeitintervallen selbsttätig eine Kalibrierung des in der Signalverarbeitungseinheit 16 verarbeiteten Ausgangssignals des Körpersensors 26 durchgeführt wird.

Während bei dem zuvor beschriebenen Ausführungsbeispiel die Fördereinheit 12 als Pumpe zur Entnahme von interstitieller Flüssigkeit vorgesehen ist, kann alternativ oder in Ergänzung auch eine Fördereinheit 60 zum Einleiten einer Kalibrierlösung oder Dosiereinheit 62 zum Einleiten einer insbesondere insulinhaltigen Medikationsflüssigkeit in das Körpergewebe an den Fluidkanal 28 angeschlossen sein. Auch hierbei lässt sich der Flüssigkeitsdurchsatz bzw. die Dosiermenge mittels der Steuereinrichtung 20 einstellen. Gegebenenfalls kann anstelle einer einfachen Steuerkette auch ein geschlossener Regelkreis für den Flüssigkeitstransport vorgesehen sein.

Abhängig von der Zusammensetzung der zugeführten Flüssigkeit kann der Abstand des Sensors 26 von der gewebeseitigen Öffnung 46 des Fluidkanals 28 unterschiedlich gewählt werden. Im Fall der Zufuhr einer Lösung bekannten Analytgehalts sollte der Abstand möglichst gering sein, so dass nach Austritt der Flüssigkeit aus der Öffnung 46 eine unmittelbare Änderung des Messsignals eintritt. Im Fall der Zufuhr einer Wirkstofflösung (Insulin), welche die Analytkonzentration (Glucose) im Gewebe beeinflusst, wird der Abstand so gewählt, dass bei gegebenem Druckunterschied zwischen Öffnung 46 und Gewebsumgebung, gegebenen kolloidosmotischen Druck, gegebener Zeitdauer der Flüssigkeitszufuhr und bei gegebenem Diffusionskoeffizienten des Analyten keine messbare Veränderung der Analytkonzentration der Körperflüssigkeit in unmittelbarer Umgebung des Sensors erfolgt.

Eine weitere, nicht eigens gezeigte Ausführungsform beinhaltet zwei Einzelsensoren für den Analyten, die im Abstand zueinander stehen, wobei einer der Einzelsensoren näher an der gewebeseitigen Öffnung 46 angeordnet ist. Dies ermöglicht eine Erfassung an zwei unterschiedlichen subkutanen Orten, die aufgrund von Inhomogenitäten des subkutanen Gewebes und der jeweiligen Gewebsreaktion vorteilhaft ist. Ferner kann bei Zufuhr von Wirkstofflösungen über den Fluidkanal 28 mittels des einen Einzelsensors eine Wechselwirkung des Wirkstoffs mit dem umgebenden Gewebe zwecks Erfassung eines Krankheitszustandes und der Therapiekontrolle bestimmt werden, während mittels des anderen Einzelsensors die Zufuhr der Wirkstofflösung gesteuert werden kann. Mit den genannten Einzelsensoren ist es auch möglich, einen Analyten und einen über den Fluidkanal 28 eingeleiteten Wirkstoff gesondert zu bestimmen. Hierbei sollte der Analytsensor einen größeren Abstand von der Öffnung 46 aufweisen als der Wirkstoffsensor. Zusätzlich zur Bestimmung der Analytkonzentration ermöglicht dies die Bestimmung des Wirkstoffs im umliegenden Gewebe und somit die Erfassung eines Krankheitzustandes und eine Therapiekontrolle.

Denkbar ist es auch, zwei Analytsensoren und einen Wirkstoffsensor vorzusehen, wobei der erste Analytsensor einen größeren Abstand von der gewebeseitigen Öffnung 46 aufweist als der zweite Analytsensor und als der Wirkstoffsensor. Diese Anordnung vereinigt die Vorteile der beiden oben beschriebenen Ausführungsformen.

## Patentansprüche

1. Sensorsystem, insbesondere zur Glucoseüberwachung, mit einem zumindest partiell in Körpergewebe, vorzugsweise Unterhautgewebe einsetzbaren Träger (24), einem an dem Träger (24) angeordneten Fluidkanal (28) zum Durchleiten einer Flüssigkeit und einem auf einen Inhaltsstoff der Flüssigkeit und/oder des Körpergewebes ansprechenden Sensor (26), wobei der Sensor (26) für einen direkten Kontakt mit dem Körpergewebe getrennt von dem Fluidkanal (28) an dem Träger (24) angeordnet ist, **dadurch gekennzeichnet, dass** eine außerhalb des Körpergewebes befindliche Kalibriereinrichtung (22) für den Sensor (26) über den Fluidkanal (28) mit interstitieller Flüssigkeit beaufschlagbar ist, wobei der Fluidkanal (28) an eine Pumpeinheit (12) zur Entnahme von interstitieller Flüssigkeit aus dem Körpergewebe angeschlossen ist und die Kalibriereinrichtung (22) einen Referenzsensor (58) zum Kalibrieren der Ausgangssignale des Sensors (26) aufweist.

2. Sensorsystem nach Anspruch 1, **dadurch gekennzeichnet, dass** der Sensor (26) im Abstand von einer gewebeseitigen Öffnung (46) des Fluidkanals (28) in Eingriff mit dem Körpergewebe steht.

3. Sensorsystem nach Anspruch 1 oder 2, **dadurch gekennzeichnet, dass** der Fluidkanal (28) an eine Dosiereiriheit (62) zum Einleiten einer insbesondere insulinhaltigen Medikationsflüssigkeit in das Körpergewebe angeschlossen ist.

4. Sensorsystem nach einem der Ansprüche 1 bis 3, **dadurch gekennzeichnet, dass** der Fluidkanal (28) an eine Fördereinheit (60) zum Einleiten einer Kalibrierlösung in das Körpergewebe angeschlossen ist.

5. Sensorsystem nach einem der Ansprüche 1 bis 4, dadurch gekennzeichet, dass der Sensor (26) einen in Berührung mit dem Körpergewebe bringbaren aktiven Sensorbereich (44) aufweist.

6. Sensorsystem nach einem der Ansprüche 1 bis 5, **dadurch gekennzeichnet, dass** der Sensor (26) zur vorzugsweise kontinuierlichen Erfassung von Glucose als Inhaltsstoff in der interstitiellen Flüssigkeit ausgebildet ist.

7. Sensorsystem nach einem der Ansprüche 1 bis 6, dadurch gekennzeichet, dass der Sensor (26) in Mehrelektrodenausführung mehrere auf einer in das Körpergewebe eingreifenden Oberfläche des Trägers (24) angeordnete Elektroden (32,34,36) aufweist.

8. Sensorsystem nach einem der Ansprüche 1 bis 7, **dadurch gekennzeichnet, dass** der Träger (24) durch ein flexibles Substrat gebildet ist, wobei der Fluidkanal (28) und der Sensor (26) in dem Substrat integriert sind.

9. Sensorsystem nach einem der Ansprüche 1 bis 8, **dadurch gekennzeichnet, dass** der vorzugsweise biegsame und stumpfkantige Träger (24) über Einführhilfsmittel in das Körpergewebe einsetzbar ist.

10. Sensorsystem nach einem der Ansprüche 1 bis 9, **dadurch gekennzeichnet, dass** der Fluidkanal (28) mindestens eine gewebeseitige Öffnung (46), mindestens eine außerhalb des Körpergewebes befindliche Öffnung (48) und mindestens einen längsseitig geschlossenen oder halboffenen, insbesondere kapillaren Fließpfad (50) zwischen den Öffnungen (46,48) aufweist.

11. Sensorsystem nach einem der Ansprüche 1 bis 10, **dadurch gekennzeichnet, dass** der Fluidkanal (28) vorzugsweise durch Laserbearbeitung, Heißprägen oder Photolithographie in den gegebenenfalls mehrteiligen Träger (24) integriert ist.

12. Sensorsystem nach einem der Ansprüche 1 bis 11, **dadurch gekennzeichnet, dass** der Fluidkanal (28) einen Durchflussquerschnitt von weniger als 1 mm² aufweist.

13. Sensorsystem nach einem der Ansprüche 1 bis 12, dadurch gekenntzeichnet, dass der Fluidkanal (28) auf einer den Sensor (26) tragenden Fläche des Trägers (24) im Erfassungsbereich des Sensors (26) mündet.

14. Sensorsystem nach einem der Ansprüche 1 bis 12, **dadurch gekennzeichnet, dass** der Fluidkanal (28) an einer von dem Sensor (26) abgewandten oder entfernten Partie des Trägers (24) außerhalb des Erfassungsbereichs des Sensors (26) mündet.

15. Sensorsystem nach einem der Ansprüche 1 bis 14, **dadurch gekennzeichnet, dass** als Sensor (26) mindestens zwei in unterschiedlichem Abstand von einer gewebeseitigen Öffnung des Fluidkanals (28) befindliche Einzelsensoren vorgesehen sind.

16. Sensorsystem nach einem der Ansprüche 1 bis 15, **dadurch gekennzeichnet, dass** der Sensor (26) einen Einzelsensor für einen Analyten und einen Einzelsensor für einen über den Fluidkanal (28) in das Körpergewebe einleitbaren Wirkstoff aufweist.

17. Anordnung zur Überwachung eines Inhaltsstoffs, insbesondere Glucose in Körpergewebe, **gekennzeichnet durch** ein Sensorsystem (10) nach einem der vorhergehenden Ansprüche.

18. Anordnung nach Anspruch 17, **dadurch gekennzeichnet, dass** das Sensorsystem (10) mit einer Signalverarbeitungseinheit (16) zur quantitativen Bestimmung des Inhaltsstoffs gekoppelt ist.

19. Anordnung nach Anspruch 17 oder 18, **dadurch gekennzeichnet, dass** die durch den Fluidkanal (28) hindurchgeleitete, insbesondere insulinhaltige Flüssigkeitsmenge mittels einer Steuer- oder Regeleinrichtung (20) kontrollierbar ist.

20. Verfahren zur Überwachung eines Inhaltsstoffs, insbesondere Glucose in Körpergewebe, bei welchem ein Träger (24) zumindest partiell in Körpergewebe, vorzugsweise Unterhautgewebe eingesetzt wird, über einen an dem Träger (24) angeordneten Fluidkanal (28) eine Flüssigkeit aus dem Körpergewebe entnommen wird und mittels eines Sensors (26) ein Inhaltsstoff der Flüssigkeit und/oder des Körpergewebes überwacht wird, wobei der Sensor (26) an dem Träger (24) getrennt von dem Fluidkanal (28) angeordnet und in direkten Kontakt mit dem Körpergewebe gebracht wird, **dadurch gekennzeichnet, dass** über den Fluidkanal (28) interstitielle Flüssigkeit entnommen und der Inhaltsstoff mittels eines Referenzsensors (58) erfasst wird, und dass der in Kontakt mit dem Körpergewebe befindliche Sensor (26) nach Maßgabe des Referenzsensors (58) kalibriert wird.

21. Verfahren nach Anspruch 20, **dadurch gekennzeichnet, dass** die Kalibrierung in gegebenen Zeitintervallen automatisch durchgeführt wird.

22. Verfahren nach einem der Ansprüche 20 oder 21, **dadurch gekennzeichnet, dass** bei Unter- oder Überschreiten eines vorgegebenen Grenzwerts der erfassten Konzentration des Inhaltsstoffs ein Alarm ausgelöst wird.

23. Verfahren nach einem der Ansprüche 20 bis 22, **dadurch gekennzeichnet, dass** bei Unterschreiten einer Mindestversorgungsspannung des Sensorsystems ein Alarm ausgelöst wird.

## Claims

1. Sensor system in particular for glucose monitoring comprising a support (24) that can be at least partially inserted into body tissue, preferably subcutaneous tissue, a fluid channel (28) arranged on the support (24) for passing through a liquid and a sensor (26) that responds to a constituent of the liquid and/or of the body tissue, wherein the sensor (26) is arranged on the support (24) separately from the fluid channel (28) for a direct contact with the body tissue, **characterized in that** interstitial fluid can be fed via the fluid channel (28) to a calibration device (22) for the sensor (26) that is located outside of the body tissue, wherein the fluid channel (28) is connected to a pumping unit (12) to remove interstitial fluid from the body tissue and the calibration device (22) has a reference sensor (58) to calibrate the output signals of the sensor (26).

2. Sensor system according to claim 1, **characterized in that** the sensor (26) is in engagement with the body tissue at a distance from an opening (46) of the fluid channel (28) on the tissue side.

3. Sensor system according to claim 1 or 2, **characterized in that** the fluid channel (28) is connected to a metering unit (62) to feed a medication liquid and in particular one containing insulin into the body tissue.

4. Sensor system according to one of the claims 1 to 3, **characterized in that** the fluid channel (28) is connected to a conveying unit (60) to feed a calibration solution into the body tissue.

5. Sensor system according to one of the claims 1 to 4, **characterized in that** the sensor (26) has an active sensor region (44) which can be brought into contact with the body tissue.

6. Sensor system according to one of the claims 1 to 5, **characterized in that** the sensor (26) is designed to preferably continuously detect glucose as a constituent in the interstitial fluid.

7. Sensor system according to one of the claims 1 to 6, **characterized in that** the sensor (26) in a multi-electrode configuration has several electrodes (32, 34, 36) arranged on a surface of the support (24) which engages with the body tissue.

8. Sensor system according to one of the claims 1 to 7, **characterized in that** the support (24) is formed by a flexible substrate wherein the fluid channel (28) and the sensor (26) are integrated in the substrate.

9. Sensor system according to one of the claims 1 to 8, **characterized in that** the preferably flexible and blunt-edged support (24) can be inserted into the body tissue by means of an insertion aid.

10. Sensor system according to one of the claims 1 to 9, **characterized in that** the fluid channel (28) has at least one opening (46) on the tissue side, at least one opening (48) located outside of the body tissue and at least one flow path (50) between the openings (46, 48) that is closed or half open on the longitudinal side and is in particular a capillary flow path (50).

11. Sensor system according to one of the claims 1 to 10, **characterized in that** the fluid channel (28) is integrated into the optionally multi-part support (24) preferably by laser treatment, hot stamping or photolithography.

12. Sensor system according to one of the claims 1 to 11, **characterized in that** the fluid channel (28) has a throughflow cross-section of less than 1 mm².

13. Sensor system according to one of the claims 1 to 12, **characterized in that** the fluid channel (28) opens onto a surface of the support (24) carrying the sensor (26) in the detection area of the sensor (26).

14. Sensor system according to one of the claims 1 to 12, **characterised in that** the fluid channel (28) opens at a part of the support (24) that faces away from or is distant from the sensor (26) and is outside of the detection area of the sensor (26).

15. Sensor system according to one of the claims 1 to 14, **characterized in that** at least two single sensors that arc at different distances from an opening of the fluid channel (28) on the tissue side are provided as a sensor (26).

16. Sensor system according to one of the claims 1 to 15, **characterized in that** the sensor (26) has a single sensor for an analyte and a single sensor for an active substance that can be passed into the body tissue through the fluid channel (28).

17. Arrangement for monitoring a constituent and in particular glucose in body tissue, **characterized by** a sensor system (10) according to one of the previous claims.

18. Arrangement according to claim 17, **characterized in that** the sensor system (10) is coupled to a signal processing unit (16) for the quantitative determination of the constituent.

19. Arrangement according to claim 17 or 18, **characterized by** the fact that the quantity of liquid and in particular insulin-containing quantity of liquid that is passed through the fluid channel (28) can be controlled by a control or regulating device (20).

20. Method for monitoring a constituent and in particular glucose in body tissue in which a support (24) is inserted at least partially into body tissue and preferably into subcutaneous tissue, a liquid is removed from the body tissue by means of a fluid channel (28) arranged on the support (24) and a constituent of the liquid and/or of the body tissue is monitored with the aid of a sensor (26), wherein the sensor (26) is arranged on the support (24) separately from the fluid channel (28) and is brought into direct contact with the body tissue, **characterized in that** interstitial fluid is removed via the fluid channel (28) and the constituent is detected by means of a reference sensor (58), and that the sensor (26) that is in contact with the body tissue is calibrated in accordance with the reference sensor (58).

21. Method according to claim 20, **characterized in that** the calibration is carried out automatically at specified time intervals.

22. Method according to one of the claims 20 or 21, **characterized in that** an alarm is triggered when the detected concentration of the constituent falls below or exceeds a preset threshold value.

23. Method according to one of the claims 20 to 22, **characterized in that** an alarm is triggered when the voltage for the sensor system falls below a minimum supply voltage.

## Revendications

1. Système de capteur, en particulier pour la surveillance du glucose, comprenant un support (24) apte à être placé au moins partiellement dans le tissu corporel, de préférence le tissu sous-cutané, un canal de fluide (28) disposé sur le support (24) permettant le passage d'un liquide et un capteur (26) répondant à un composant du liquide et/ou du tissu corporel, ledit capteur (26), de manière à assurer un contact direct avec le tissu corporel, étant disposé sur le support (24) à l'écart du canal de fluide (28), **caractérisé en ce qu'**un dispositif de calibrage (22) pour le capteur (26) situé à l'extérieur du tissu corporel peut être alimenté en liquide interstitiel à travers le canal de fluide (28), ledit canal de fluide (28) étant connecté à une unité de pompage (12) pour le prélèvement de liquide interstitiel dans le tissu corporel, et ledit dispositif de calibrage (22) comportant un capteur de référence (58) pour le calibrage des signaux de sortie du capteur (26).

2. Système de capteur selon la revendication 1, **caractérisé en ce que** le capteur (26) est en prise avec le tissu corporel, à distance d'un orifice (46) coté tissu du canal de fluide (28).

3. Système de capteur selon la revendication 1 ou 2, **caractérisé en ce que** le canal de fluide (28) est connecté à une unité de dosage (62) permettant d'introduire un liquide médicamenteux, contenant notamment de l'insuline, dans le tissu corporel.

4. Système de capteur selon l'une des revendications 1 à 3, **caractérisé en ce que** le canal de fluide (28) est connecté à une unité de transport (60) permettant d'introduire une solution de calibrage dans le tissu corporel.

5. Système de capteur selon l'une des revendications 1 à 4, **caractérisé en ce que** le capteur (26) comporte une zone active (44) de capteur pouvant être mise en contact avec le tissu corporel.

6. Système de capteur selon l'une des revendications 1 à 5, **caractérisé en ce que** le capteur (26) est conçu pour la détection, de préférence continue, du glucose comme composant dans le liquide interstitiel.

7. Système de capteur selon l'une des revendications 1 à 6, **caractérisé en ce que** le capteur (26) de type multi-électrodes comporte plusieurs électrodes (32, 34, 36) disposées sur une surface du support (24) pénétrant dans le tissu corporel.

8. Système de capteur selon l'une des revendications 1 à 7, **caractérisé en ce que** le support (24) est formé par un substrat flexible, le canal de fluide (28) et le capteur (26) étant intégrés dans le substrat.

9. Système de capteur selon l'une des revendications 1 à 8, **caractérisé en ce que** le support (24), de préférence souple et aux bords émoussés, peut être placé dans le tissu corporel à l'aide d'instruments d'introduction.

10. Système de capteur selon l'une des revendications 1 à 9, **caractérisé en ce que** le canal de fluide (28) comporte au moins un orifice (46) côté tissu, au moins un orifice (48) situé à l'extérieur du tissu et au moins une voie d'écoulement (50) longitudinale fermée ou semi-ouverte, en particulier capillaire, entre les orifices (46, 48).

11. Système de capteur selon l'une des revendications 1 à 10, **caractérisé en ce que** le canal de fluide (28) est intégré de préférence par façonnage au laser, estampage à chaud ou photolithographie dans le support (24), réalisé le cas échéant en plusieurs pièces.

12. Système de capteur selon l'une des revendications 1 à 11, **caractérisé en ce que** le canal de fluide (28) présente une section de passage inférieure à 1 mm².

13. Système de capteur selon l'une des revendications 1 à 12, **caractérisé en ce que** le canal de fluide (28) débouche, sur une surface du support (24) portant le capteur (26), dans la zone de détection du capteur (26).

14. Système de capteur selon l'une des revendications 1 à 12, **caractérisé en ce que** le canal de fluide (28) débouche, sur une partie du support (24) opposée au ou éloignée du capteur (26), à l'extérieur de la zone de détection du capteur (26).

15. Système de capteur selon l'une des revendications 1 à 14, **caractérisé en ce qu'**il est prévu comme capteur (26), au moins deux capteurs individuels situés à des distances différentes d'un orifice côté tissu du canal de fluide (28).

16. Système de capteur selon l'une des revendications 1 à 15, **caractérisé en ce que** le capteur (26) comporte un capteur individuel pour un analyte et un capteur individuel pour un principe actif pouvant être introduit dans le tissu corporel à travers le canal de fluide (28).

17. Dispositif pour surveiller un composant, en particulier le glucose dans le tissu corporel, **caractérisé par** un système de capteur (10) selon l'une des revendications précédentes.

18. Dispositif selon la revendication 17, **caractérisé en ce que** le système de capteur (10) est couplé à une unité de traitement du signal (16) permettant la quantification du composant.

19. Dispositif selon la revendication 17 ou 18, **caractérisé en ce que** la quantité de liquide passant à travers le canal de fluide (28), contenant notamment de l'insuline, peut être contrôlée au moyen d'un dispositif de commande ou de régulation (20).

20. Procédé pour surveiller un composant, en particulier le glucose dans le tissu corporel, consistant à placer un support (24) au moins partiellement dans le tissu corporel, de préférence le tissu sous-cutané, à prélever un liquide dans le tissu corporel à travers un canal de fluide (28) disposé sur le support (24), et à surveiller un composant du liquide et/ou du tissu corporel au moyen d'un capteur (26), lequel capteur (26) est placé sur le support (24) à l'écart du canal de fluide (28) et est mis en contact direct avec le tissu corporel, **caractérisé en ce que** l'on prélève du liquide interstitiel à travers le canal de fluide (28) et l'on détecte le composant au moyen d'un capteur de référence (58), et **en ce que** le capteur (26) en contact avec le tissu corporel est calibré selon le capteur de référence (58).

21. Procédé selon la revendication 20, **caractérisé en ce que** le calibrage est effectué automatiquement à des intervalles spécifiés.

22. Procédé selon l'une des revendications 20 ou 21, **caractérisé en ce qu'**une alarme est déclenchée lorsque la concentration détectée du composant est inférieure ou supérieure à une valeur limite prédéfinie.

23. Procédé selon l'une des revendications 20 à 22, **caractérisé en ce qu'**une alarme est déclenchée lorsque le système de capteur descend au-dessous d'une tension d'alimentation minimale.
